# FASCICULE DE BREVET EUROPEEN

(11) **EP 0 687 283 B1**
(45) Date de publication et mention de la délivrance du brevet: **17.05.2000**
(21) Numéro de dépôt: 95904590.7
(22) Date de dépôt: 22.12.1994
(51) Int. Cl.: C08J 5/18, C08K 5/00, C08J 3/09, C08J 3/215, C08L 21/00, A61F 6/00, A61B 19/04, A01N 25/34

(54) **FILM D'ELASTOMERE COMPRENANT DES GOUTTELETTES, SON PROCEDE DE PREPARATION ET SES APPLICATIONS**
TRÖPFCHEN ENTHALTENDE ELASTOMERFOLIE , IHR HERSTELLUNGSVERFAHREN UND IHRE VERWENDUNG
ELASTOMERIC FILM COMPRISING DROPLETS, METHOD FOR PREPARING SAME, AND USES THEREOF

(30) Priorité: 23.12.1993 FR 9315561
(43) Date de publication de la demande: 20.12.1995
(73) Titulaire: HUTCHINSON, 75008 Paris (FR)
(72) Inventeur: BUSNEL, René, Guy, F-91570 Bièvres (FR); CHEYMOL, André, F-86220 Dange-Saint-Romain (FR); RIESS, Gérard, F-68200 Mulhouse (FR)
(74) Mandataire: Ores, Irène
(86) Numéro de dépôt international: FR9401515
(87) Numéro de publication internationale: WO9517453

(56) Documents cités:
- EP-A- 0 141 628
- EP-A- 0 306 389
- EP-A- 0 338 821
- EP-A- 0 555 116
- WO-A-93/02668
- AU-B- 459 228
- DE-A- 1 494 024
- DE-A- 3 035 851
- FR-A- 2 600 656
- GB-A- 372 776
- GB-A- 1 179 497
- GB-A- 2 263 114
- NL-A- 7 308 061
- US-A- 2 947 282
- US-A- 4 725 575
- DATABASE WPI Week 8326, Derwent Publications Ltd., London, GB; AN 83-61887K & JP,A,57 185 326 (KURARAY KK) 15 November 1982
- Encyclopedia of Polymer Science and Engineering, vol. 9, "Microencapsulation", John Wiley and Sons, New York, 1987, pages 724-725

## Description

La présente invention est relative à des films d'élastomère, dans lesquels sont dispersées uniformément, sous la forme de gouttelettes de liquide, des substances chimiques actives, telles que des anti-corrosifs, des lubrifiants ou encore des biocides à usage médical ; elle couvre également les procédés de préparation ainsi que les diverses applications de ces films.

La présente invention est également relative à des émulsions stables comportant une phase continue formée d'une solution d'élastomère dans un solvant organique et une phase dispersée comprenant une substance chimique active dans un solvant non miscible avec la solution d'élastomère, aptes à être transformées en films d'élastomère, ainsi qu'à leur procédé de préparation.

Les différents matériaux élastomères, habituellement utilisés dans le domaine médical ou paramédical (hygiène, notamment) peuvent être modifiés, de manière à être associés à des substances chimiques actives, ayant un effet de protection, lors de l'utilisation de ces matériaux (gants, doigtiers, préservatifs, bandes et pansements divers).

En effet, aussi bien dans les cas d'examen ou d'intervention chirurgicale ou en odontologie, que pour la protection à l'encontre d'agents pathogènes tels que, par exemple, bactéries, virus et spores fongiques, une rupture ou même parfois simplement les pores ou une fissure de la membrane d'élastomère, peut entraîner une contamination du porteur dudit matériau, dont l'utilisation n'est ainsi pas sans risque d'autant plus que dans les pratiques de ces métiers, des piqûres de seringues, d'aiguilles à suture, de trocard, d'éclats d'os, etc, interviennent relativement que fréquemment.

Différents traitements ont été proposés pour tenter de réduire la marge de risque lors de l'utilisation de produits fabriqués avec ces matériaux élastomères :
- la dispersion d'une substance active sous forme liquide dans l'élastomère, en tant que plastifiant de ce dernier ; toutefois, dans ce cas, le liquide est peu disponible en tant que tel, puisqu'il est lié à l'élastomère. En outre, dans le cas où une partie de cette substance chimique n'est pas liée à l'élastomère, le liquide contenant ladite substance chimique, non liée, peut conduire à une séparation de phase macroscopique et à des phénomènes de migration au cours du temps ; en outre, le liquide se concentre dans certaines zones, laissant ainsi toute une surface de l'élastomère sans substance active.
- le liquide peut être incorporé entre deux gaines d'élastomère non solidarisées ; dans ce cas, il ne joue plus le râle d'un plastifiant et présente une plus grande disponibilité. Toutefois, un tel traitement a l'inconvénient de mettre la substance active en contact direct avec l'élastomère, ce qui agit sur ce dernier en détruisant ses propriétés mécaniques et donc, par conséquent, son imperméabilité. En outre, le liquide contenu entre les deux gaines a tendance, lors de frottements, à se rassembler en un seul endroit, laissant également toute une surface du matériau sans substance active (Brevet US 2,586,674).
- pour pallier ces inconvénients, la Demanderesse a introduit dans un matériau élastomère, un liquide microencapsulé, soit entre deux couches en matériau élastomère (Demande EP 306 389), soit dans le matériau élastomère (Demande internationale WO 93/02668). L'utilisation de microcapsules rend le liquide effectivement disponible en tant que tel ; toutefois, les parois des microcapsules peuvent se révéler difficiles à percer et modifient, dans certains cas, les propriétés mécaniques de l'élastomère.

En conséquence, poursuivant ses recherches, la Demanderesse s'est donné pour but la mise au point d'un matériau élastomère qui ne présente pas les inconvénients des matériaux chargés en substance chimique active de l'Art antérieur, notamment:
. en ce que le liquide contenant la substance chimique active est disponible aussi bien en cas de déchirure que par simple frottement, sous forme de fines gouttelettes stabilisées, de manière uniforme dans l'ensemble dudit matériau, le liquide ne se concentrant pas dans certaines zones de ce dernier, et
. en ce que les propriétés mécaniques dudit matériau ne sont pas modifiées.

La présente invention a pour objet un film d'élastomère comprenant une substance chimique active sous forme liquide, lequel film est caractérisé en ce qu'il comprend :
- une dispersion de gouttelettes consistant en une solution chimique active dans un polyol, d'une substance chimique active sélectionnée dans le groupe constitué par les anticorrosifs, les lubrifiants et les biocides, et
- un copolymère de stabilisation (bloc ou greffé) de ladite dispersion de gouttelettes comportant au moins des séquences poly B, miscibles avec lesdites gouttelettes, et des séquences poly A, non miscibles avec ces gouttelettes, ledit copolymère étant sélectionné dans le groupe constitué par les copolymères di-bloc, de type poly A-bloc-poly B, les copolymères tri-bloc sélectionnés dans le groupe constitué par les copolymères poly B-bloc-poly A-bloc-poly B (BAB), poly A-bloc-poly B-bloc-poly A (ABA), poly A-bloc-poly B-bloc-poly C (ABC) et poly A-bloc-poly C-bloc-poly B (ACB) et les copolymères greffés de type poly A-greffé-poly B, poly B-greffé-poly A, de type poly A-greffé-poly B et poly C ou de type poly C-greffé-poly A et poly B.

Selon un mode de réalisation avantageux dudit film d'élastomère, ledit polyol ou solvant b est sélectionné dans le groupe constitué par le polypropylène glycol, le polyéthylène glycol et le glycérol et un mélange de ceux-ci.

Selon un autre mode de réalisation avantageux dudit film d'élastomère, les proportions de séquences poly A sont comprises entre 10 et 90 % et les proportions de séquences poly B sont comprises entre 90 et 10 % (par rapport à la somme des séquences poly A + séquences poly B) et les proportions de séquences poly C sont comprises entre 0 % et 50 % (par rapport au total de séquences).

Selon encore un autre mode de réalisation avantageux dudit film d'élastomère, les masses moléculaires des séquences poly A et poly B sont comprises entre 1 000 et 500 000 Daltons.

Conformément à l'invention, **les séquences poly A** sont choisies dans le groupe constitué par les polydiènes, les polyoléfines, les polyéthers ou les silicones, miscibles avec une solution d'élastomère dans un solvant a apolaire ou peu polaire et non-miscibles avec le polyol (ou solvant b) et **les séquences poly B,** miscibles avec le polyol sont choisies dans le groupe qui comprend le polyoxyéthylène, la polyvinylpyrrolidone, les polyacides acryliques, le poly(alcool vinylique) et le poly(vinylpyridine) quaternisé.

Selon une disposition avantageuse de ce mode de réalisation, les séquences poly A sont sélectionnées dans le groupe constitué par le polyisoprène, le polybutadiène, le polyisobutène, le polybutadiène hydrogéné ou le polyisoprène hydrogéné, le polystyrène, le polytertiobutylstyrène, le polyoxypropylène, le polydiméthylsiloxane.

Parmi les structures greffées comportant des séquences poly A, poly B et poly C, on peut notamment citer le copolymère Goldschmidt LE 2229® qui correspond à une structure greffée où :
- les séquences poly C sont des chaînes acryliques, par exemple,
- les séquences poly A sont des chaînes alkyles, et
- les séquences poly B sont des séquences poly(oxyéthylène).

Dans de tels copolymères, les séquences poly A sont des séquences miscibles avec la solution d'élastomère (solvant a apolaire) ; les séquences poly B sont miscibles avec le solvant b (polyol) et les séquences poly C peuvent être soit miscibles avec le solvant a ou le solvant b, soit non miscible avec les solvants a et b.

L'élastomère peut être sélectionné, et ce, de manière non limitative, parmi le polybutadiène, le polyisoprène, le polychloroprène, les copolymères SBR *(Styrene Butadiene Rubber)*, NBR *(Nitrile Butadiene Rubber)*, SBS *(Styrene Butadiene Styrene)*, SIS *(Styrene Isoprene Styrene)* ou SEBS, de masse moléculaire de préférence supérieure à 50 000.

Un tel film d'élastomère, dans lequel le liquide, contenant la ou les substances chimiques actives, est dispersé, de manière uniforme et stable, sous la forme de fines gouttelettes, rend immédiatement disponible et efficace ladite ou lesdites substances actives incluses dans le matériau élastomère, même en cas de frottement (absence de couche intermédiaire ou de parois de microcapsules). En effet, les gouttelettes de liquide, d'un diamètre compris entre 0,1 à 100 µm, sont suffisamment grandes pour fournir du liquide en quantité suffisante au moment opportun.

Selon un autre mode de réalisation avantageux dudit film d'élastomère, la substance chimique active est choisie parmi les biocides.

Selon une disposition avantageuse de ce mode de réalisation, ledit biocide est sélectionné dans le groupe constitué par les ammonium quaternaires, les copolymères bloc à activité biocide, les biguanides, le phtaraldéhyde, les dérivés phénoliques à activité biocide, les tensioactifs non ioniques comportant une séquence polyoxyéthylène ou l'hexamidine et leur mélange.

En variante, le copolymère bloc intervient à la fois en tant qu'agent de stabilisation de l'émulsion et en tant que substance active.

De manière préférée, un tel copolymère bloc est avantageusement du PMDS-POE (activité biocide), à savoir copolymère bloc de polydiméthylsiloxane-polyoxyéthylène.

Selon encore un autre mode de réalisation avantageux dudit film d'élastomère, la substance chimique active est choisie dans le groupe constitué par les anticorrosifs et les lubrifiants solubles dans les polyols.

Le film d'élastomère selon l'invention, qui peut avantageusement servir de revêtement de supports notamment en élastomère ou de surmoulage d'un joint frottant, présente, en particulier les avantages suivants :
- dans le domaine médical et paramédical : les gants, doigtiers, préservatifs ou pansements revêtus d'un film d'élastomère conforme à l'invention présentent un gain de fiabilité et de sûreté ;
- dans l'industrie : des revêtements en film d'élastomère conforme à l'invention peuvent avantageusement être utilisés dans toutes les industries où il est souhaitable qu'une substance chimique soit libérée par frottement, notamment dans l'industrie automobile où de tels revêtements peuvent constituer un surmoulage d'un joint frottant.

La présente invention a également pour objet un procédé préparation d'un film d'élastomère, caractérisé en ce qu'il comprend :
(a) la préparation d'une émulsion qui comprend :
   - la préparation d'une phase A par dissolution de l'élastomère dans un solvant organique a ;
   - la préparation d'une phase B, par mélange d'au moins une substance chimique active sélectionnée dans le groupe constitué par les anticorrosifs, les lubrifiants et les biocides, dans un solvant organique b consistant en un polyol, non miscible avec la phase A ;
   - l'addition à la phase A ou à la phase B, dans des proportions de 0,1 à 50 %, de préférence de 0,1 à 25 %, d'un copolymère bloc ou greffé de stabilisation, sélectionné dans le groupe constitué par les copolymères di-bloc, de type poly A-bloc-poly B, les copolymères tri-bloc sélectionnés dans le groupe constitué par les copolymères poly B-bloc-poly A-bloc-poly B (BAB), poly A-bloc-poly B-bloc-poly A (ABA), poly A-bloc-poly B-bloc-poly C (ABC) et poly A-bloc-poly C-bloc-poly B (ACB) et les copolymères greffés de type poly A-greffé-poly B, poly B-greffé-poly A, de type poly A-greffé-poly B et poly C ou de type poly C-greffé-poly A et poly B, dans lesquels les séquences poly A sont miscibles avec la phase A et sont sélectionnées dans le groupe constitué par les polydiènes, les polyoléfines, les polyéthers et les silicones, les séquences poly B sont miscibles avec la phase B et sont sélectionnées dans le groupe constitué par le polyoxyéthylène, la polyvinylpyrrolidone, les polyacides acryliques, le poly(alcool vinylique) et le poly(vinylpyridine) quaternisé et lesdites séquences poly C sont soit miscibles avec le solvant a, soit avec le polyol, soit non miscibles avec le solvant a et le polyol (solvant b);
   - la dispersion de la phase B dans la phase A, pour l'obtention d'une émulsion dans laquelle la phase A constitue la phase continue et la phase B, la phase dispersée ; et
(b) l'évaporation du solvant organique a, pour l'obtention d'un film d'élastomère renfermant sous forme de dispersion stable, des gouttelettes d'une substance chimique active dans un polyol.

Les séquences poly A doivent être miscibles avec la phase A (solution d'élastomère dans le solvant a) et non-miscible avec la phase B, alors qu'elles peuvent être miscibles ou non avec l'élastomère ; les séquences poly B sont miscibles uniquement et sélectivement avec la phase B.

Cette règle de miscibilité sélective est nécessaire pour obtenir la stabilité et pour pouvoir ajuster la taille des particules de phase dispersée (phase B) de l'émulsion de départ formée d'une phase hydrophile (ou phase polaire) et d'une phase hydrophobe (ou phase apolaire) qui sert à la préparation du film, par évaporation du solvant organique a.

De manière avantageuse, la solution d'élastomère est réalisée par dissolution d'un élastomère, tel que défini ci-dessus (polybutadiène, polyisoprène, polychloroprène, copolymères SBR, NBR, SBS, SEBS ou SIS) dans un solvant organique apolaire ou peu polaire a choisi dans le groupe constitué par les hydrocarbures aromatiques, aliphatiques et alicycliques, par exemple des hydrocarbures paraffiniques, le cyclohexane, le benzène, le toluène, le xylène, la tétraline, la décaline ou un mélange de ceux-ci.

Egalement de manière avantageuse, la phase B est réalisée par mélange d'une substance active dans un polyol sélectionné dans le groupe constitué par le polypropylène glycol, le polyéthylène glycol et le glycérol ou des mélanges de ceux-ci.

Lorsque la substance chimique active est choisie parmi les complexes moléculaires de la classe des antiseptiques, elle appartient, de préférence au groupe constitué par les ammoniums quaternaires, en particulier le diméthyldidécylammonium, des copolymères bloc à activité biocide, tel que le copolymère bloc polydiméthylsiloxane-polyoxyéthylène, les biguanides (sels hydrosolubles de la chlorhexidine tels que par exemple le digluconate de chlorhexidine), le phtaraldéhyde, les dérivés phénoliques (hexachlorophène), les tensioactifs non ioniques comportant une séquence polyoxyéthylène, tel que octoxynol (Triton® X100), l'hexamidine, utilisés seuls ou en mélange, notamment mélange diméthyldidécylammonium-copolymère bloc PMDS-POE ou l'un des mélanges d'antiseptiques, tels que décrits dans la Demande de Brevet EP 0 555 116 (ammonium quaternaire-sel hydrosoluble de chlorhexidine, antiseptique non ionique-hexamidine, ammonium quaternaire-antiseptique non ionique, antiseptique non-ionique-sel hydrosoluble de chlorhexidine).

Selon un autre mode de mise en oeuvre avantageux dudit procédé, les proportions de séquences poly A sont comprises entre 10 et 90 % et les proportions de séquences poly B sont comprises entre 90 et 10 % et les proportions de séquences poly B sont comprises entre 90 et 10 % (par rapport à la somme séquences poly A + séquences poly B) et les proportions de séquences poly C sont comprises entre 0 % et 50 % (par rapport au total de séquences).

Selon un autre mode de mise en oeuvre avantageux dudit procédé, les masses moléculaires des séquences poly A et poly B sont comprises entre 1 000 et 500 000 Daltons.

Les séquences poly A et poly B sont telles que définies ci-dessus.

La présente invention a également pour objet une émulsion apte à être utilisée pour la préparation d'un film d'élastomère, caractérisée en ce qu'elle comprend :
- une phase A comprenant un élastomère dissous dans un solvant organique a, (phase hydrophobe ou apolaire), dans laquelle est dispersée une phase B comprenant au moins une substance chimique active sélectionnée dans le groupe constitué par les anticorrosifs, les lubrifiants et les biocides, en solution ou dispersée dans un solvant b (phase hydrophile ou polaire) consistant en un polyol non miscible avec la phase A,
- et un copolymère bloc ou greffé sélectionné dans le groupe constitué par les copolymères di-bloc, de type poly A-bloc-poly B, les copolymères tri-bloc sélectionnés dans le groupe constitué par les copolymères poly B-bloc-poly A-bloc-poly B (BAB), poly A-bloc-poly B-bloc-poly A (ABA), poly A-bloc-poly B-bloc-poly C (ABC) et poly A-bloc-poly C-bloc-poly B (ACB) et les copolymères greffés de type poly A-greffé-poly B, poly B-greffé-poly A, de type poly A-greffé-poly B et poly C ou de type poly C-greffé-poly A et poly B, dans lesquels les séquences poly A sont miscibles avec la phase A et sont sélectionnées dans le groupe constitué par les polydiènes, les polyoléfines, les polyéthers et les silicones, les séquences poly B sont miscibles avec la phase B et sont sélectionnées dans le groupe constitué par le polyoxyéthylène, la polyvinylpyrrolidone, les polyacides acryliques, le poly(alcool vinylique) et le poly(vinylpyridine) quaternisé et lesdites séquences poly C sont soit miscibles avec le solvant a, soit avec le polyol, soit non miscibles avec le solvant a et le polyol.

De telles émulsions de type phase hydrophile-phase hydrophobe, contenant un tel copolymère bloc ou greffé, qui joue le rôle de stabilisant de l'émulsion, sont particulièrement stables.

Le solvant a, le copolymère bloc ou greffé et l'élastomère sont tels que définis ci-dessus.

La présente invention a également pour objet un procédé de préparation desdites émulsions stables, caractérisé en ce qu'il comprend :
- la préparation d'une phase A par dissolution de l'élastomère dans un solvant organique a ;
- la préparation d'une phase B par mélange d'au moins une substance chimique active dans un solvant organique b constitué par un polyol non miscible avec la phase A ;
- l'addition à la phase A ou à la phase B, dans des proportions de 0,1 à 50 %, de préférence de 0,1 à 25 %, d'un copolymère bloc ou greffé, comportant au moins des séquences poly A, miscibles avec la phase A et des séquences poly B, miscibles avec la phase B ; et
- la dispersion de la phase B dans la phase A pour l'obtention d'une émulsion dans laquelle la phase A constitue la phase continue et la phase B, la phase dispersée.

La présente invention a également pour objet les différentes applications du film d'élastomère selon l'invention en tant que revêtement de supports (élastomères, plastiques...).

Outre les dispositions qui précèdent, l'invention comprend encore d'autres dispositions, qui ressortiront de la description qui va suivre, qui se réfère à des exemples de mise en oeuvre du procédé objet de la présente invention, avec référence aux dessins annexés, dans lesquels :
- la figure 1 représente la structure de l'émulsion et du film conforme à l'invention,
- les figures 2 à 5 illustrent des diagrammes ternaires solvant a/solvant b/substance bioactive.

Il doit être bien entendu, toutefois, que ces exemples sont donnés uniquement à titre d'illustration de l'objet de l'invention, dont ils ne constituent en aucune manière une limitation.

### EXEMPLE 1 :

### a) Préparation d'une émulsion stable conforme à l'invention :

### - Préparation de la phase continue (phase A) :

On dissout, sous agitation, du polyisoprène (PI) de synthèse de masse moléculaire comprise entre 1 000 000 et 2 000 000 dans du cyclohexane, de manière à obtenir une solution à 7,5 % en polyisoprène.

### - Préparation de la phase dispersée (phase B) :

On dissout sous agitation, du Bardac® (chlorure de diméthyldidécyl-ammonium) dans du glycérol, de manière à obtenir une solution à 10,8 % en Bardac®.

### - Addition de copolymère stabilisant :

Un copolymère tribloc POE-PI-POE (de type BAB), c'est-à-dire poly(oxyéthylène)-bloc-polyisoprène-bloc-poly(oxyéthylène) a été synthétisé par polymérisation anionique. Sa teneur pondérale en PI (déterminée par RMN H¹) est de 50,1 % ; la masse moléculaire moyenne en nombre du PI est de 78 000 (déterminée par GPC) et sa masse moléculaire totale est de 156 000.

Ce copolymère solubilisé dans le cyclohexane est rajouté à la solution de polyisoprène (phase A) à raison de 2 % en masse par rapport au polyisoprène.

### - Préparation de l'émulsion :

A la phase A, on ajoute sous agitation à température ambiante 22,3 % de phase B (glycérol + Bardac®) par rapport au PI.

On obtient ainsi une émulsion stable dont la taille de la phase dispersée est en moyenne de 3 µm.

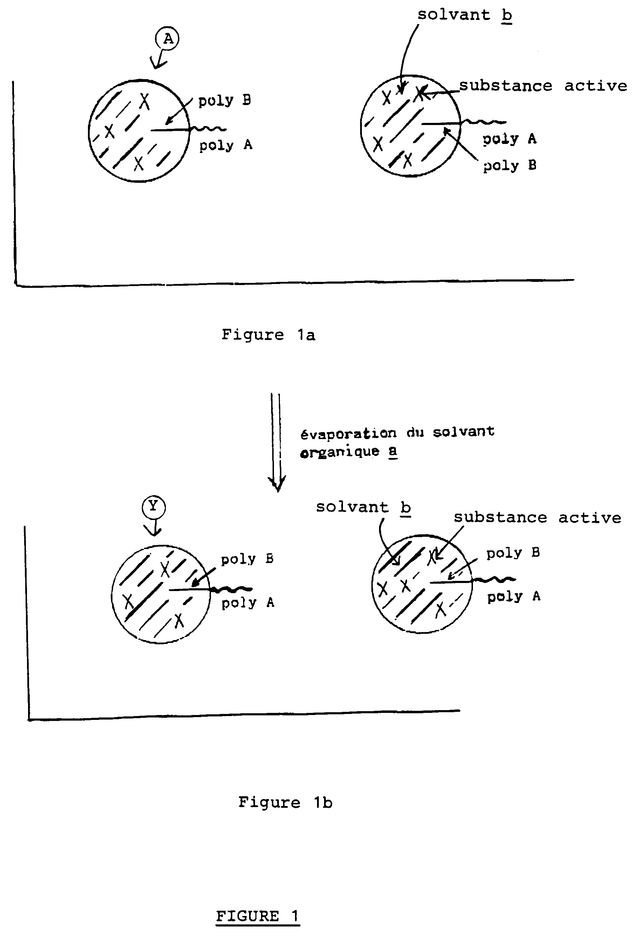

La structure est illustrée par la figure 1a, dans laquelle, Ⓐ représente la phase A continue (élastomère en solution dans le solvant organique a), /// représente la phase B dispersée (glycérol), xxx représente le biocide solubilisé dans ladite phase B et ∼∼∼― représente le copolymère bloc jouant le rôle de stabilisant de l'émulsion.

### b) Préparation d'un film d'élastomère :

On évapore le cyclohexane contenu dans l'émulsion obtenue en a) à température ambiante et sous pression atmosphérique. La structure est illustrée à la figure 1b, dans laquelle la phase B et le biocide sont représentés de la même manière qu'à la figure 1a et Ⓨ représente le film d'élastomère.

On obtient un élastomère renfermant des gouttelettes dont la taille est en moyenne de 3 µm. Ces gouttelettes renfermant la substance active représentent 22,3 % en masse du matériau total. Elles sont dispersées de manière uniforme dans l'élastomère qui conserve d 'excellentes propriétés élastiques.

### EXAMPLE 2 :

On opère comme pour l'exemple 1, en utilisant également les mêmes composants.

On augmente cependant la proportion de phase B en la portant de 22,3 % à 42,2 % par rapport au PI.

La teneur en copolymère POE-PI-POE est portée de 2 à 4,15 % par rapport au PI.

Dans ces conditions, on obtient une émulsion stable dont la taille de la phase dispersée est en moyenne de 4 µm. L'élastomère obtenu après évaporation du solvant renferme des gouttelettes de glycérol + Bardac® dont la taille est de l'ordre de 4 µm.

### EXEMPLE 3 :

On opère comme dans l'exemple 2, en remplaçant le copolymère POE-PI-POE par un copolymère di-bloc PI-POE, dont les caractéristiques sont les suivantes :
- masse moléculaire moyenne en nombre de la séquence PI : 44 000,
- pourcentage massique en PI du copolymère : 47 %,
- masse moléculaire totale : 94 000.

Ce copolymère, solubilisé dans un mélange cyclohexane-tétrahydrofuranne (2:1 en volume), est rajouté à la solution de polyisoprène à raison de 4,3 % en masse par rapport au polyisoprène.

Avec une proportion de phase B de 42,5 % par rapport au PI, on obtient une émulsion stable dont la taille de la phase dispersée est en moyenne de 8 µm.

Le film d'élastomère obtenu après évaporation du solvant, renferme des gouttelettes de glycérol + Bardac® dont la taille est de l'ordre de 8 µm.

### EXEMPLE 4 :

a) On opère comme dans l'exemple 2, en remplaçant le copolymère POE-PI-POE par un copolymère greffé polydiméthylsiloxane-poly(oxyéthylène) ayant une teneur massique en POE de 77,3 % et en PDMS de 22,7 %. Ce copolymère est commercialisé par la société GOLDSCHMIDT (Allemagne) sous la dénomination TEGOPREN 5843®. Il est engagé à raison de 5,6 % par rapport au PI.
   Comme précédemment, on obtient une émulsion stable qui, après évaporation du cyclohexane, conduit à un matériau renfermant des gouttelettes de glycérol + Bardac® dont la taille est de l'ordre de 6 µm.
   Du fait de la présence d'un constituant de type silicone, ce matériau présente en plus d'intéressantes propriétés lubrifiantes de surface.
   Ce copolymère joue le rôle d'agent stabilisant de l'émulsion. En plus, du fait de sa teneur élevée en POE, il s'agit d'un lubrifiant partiellement soluble dans le solvant b (glycérol + Bardac®).
b) On peut obtenir toute une série de films conformes à l'invention, en opérant comme dans a) mais en faisant varier la concentration en TEGOPREN 5843® de 5,6 à 40 %, par rapport au PI.

### EXEMPLE 5 :

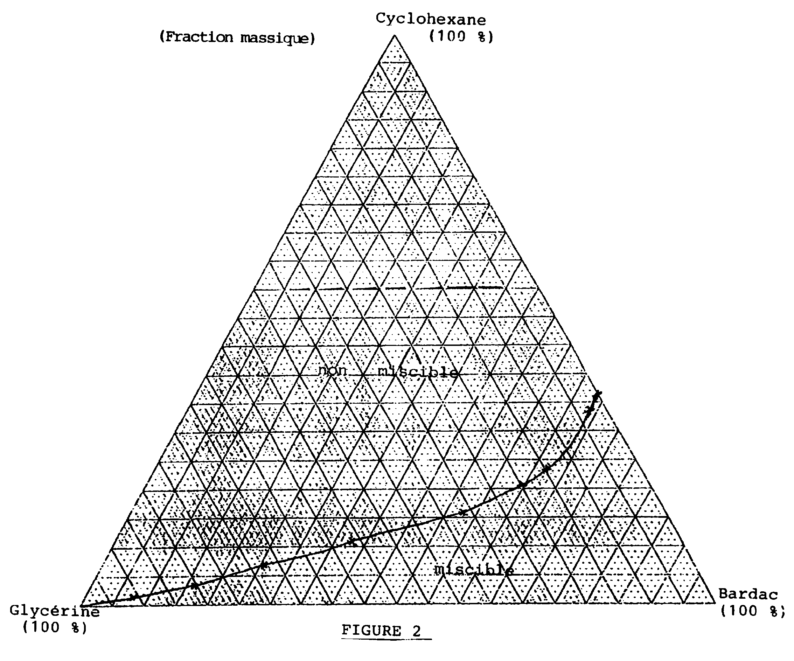

Dans les exemples ci-après, on opère comme à l'exemple 1, en faisant varier les concentrations en substances bioactives et en solvant b, tout en respectant les figures 2 à 5 (diagrammes ternaires), c'est-à-dire, en choisissant des concentrations correspondant au domaine non miscible desdits diagrammes ternaires.

La figure 2 illustre le diagramme ternaire :
cyclohexane (solvant a, apolaire)/glycérine (solvant b (polaire))/substance bioactive : Bardac®.

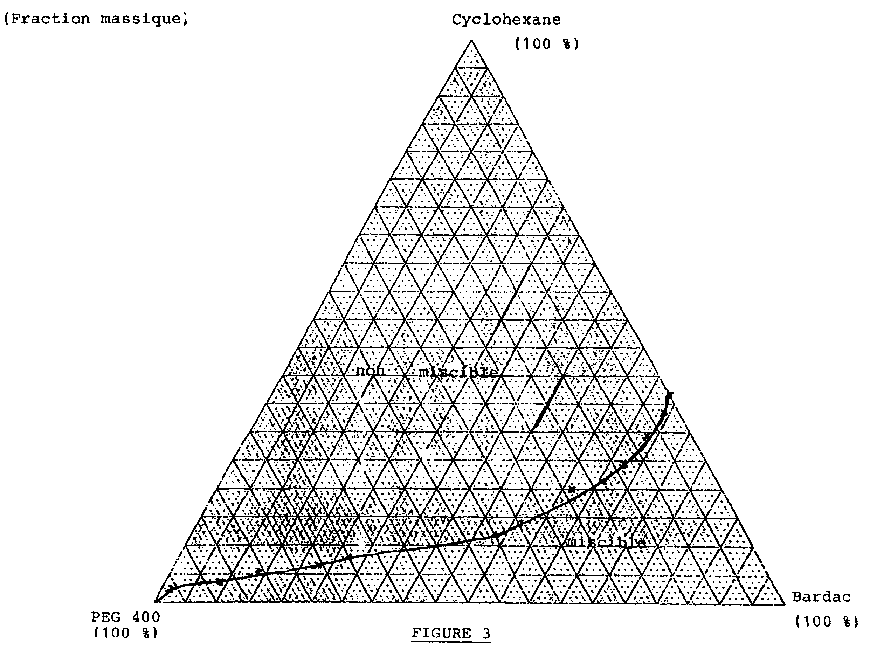

La figure 3 illustre le diagramme ternaire :
cyclohexane/PEG400/Bardac®.

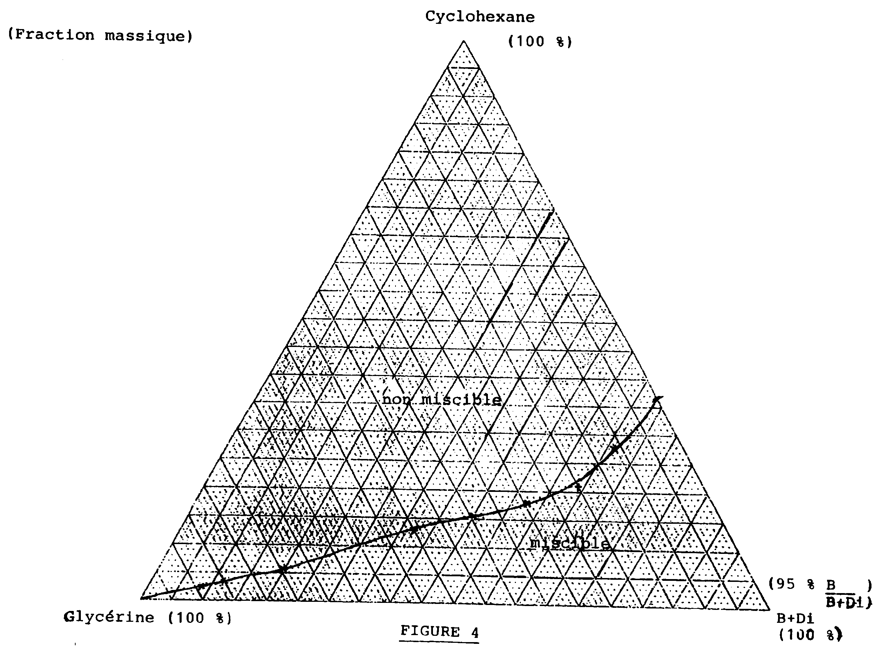

La figure 4 illustre le diagramme ternaire :
cyclohexane/glycérine/Bardac®+ digluconate de chlorhexidine (avec 95 % de Bardac®).

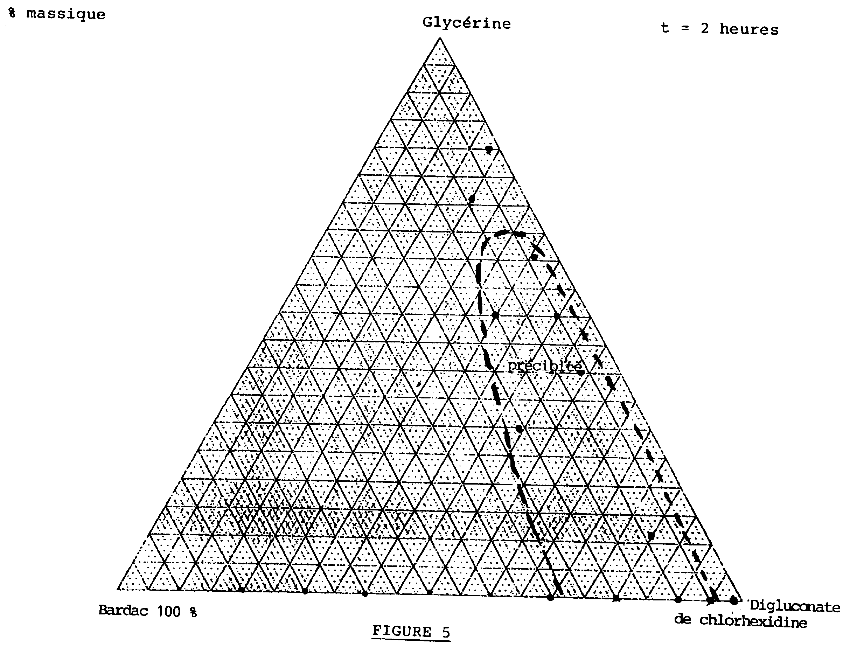

La figure 5 illustre le diagramme ternaire :
glycérine/Bardac®/digluconate de chlorhexidine.

De manière générale, l'émulsion de départ présente l'une des compositions selon le Tableau ci-après :

| Phase continue A (SEBS ou PI+solvant a) | Phase dispersée (biocides+ solvant b) | Copolymère |
|---|---|---|
| 5 % à 20 % dans un mélange cyclohexane+ xylène par exemple : | 30 à 70 % de la quantité d'élastomère (SEBS ou PI) | 1,5 à 20 %/phase continue A |
| - solvant a comprenant cyclohexane (506 parties en poids) et xylène (131 parties en poids) | | |
| - élastomère SEBS ou PI 100 parties en poids | | |

La composition de la phase dispersée varie de 10 parties de biocides + 90 parties de solvant b (PEG ou glycérol) à 50 parties de biocides + 50 parties de solvant b (PEG), avec par exemple, pour ce qui concerne les biocides :
. 90 % de Bardac® 2270 E à 70 % de principe actif,
   + 10 % de digluconate de chlorhexidine ou
. 80 % de Bardac® 2270 E à 70 % de principe actif,
   + 20 % de digluconate de chlorhexidine ou
. 75 % de Bardac® tel que défini ci-dessus,
   + 25 % de digluconate de chlorhexidine,
en respectant le diagramme ternaire selon la figure 5.

### EXEMPLE 6 : Préparation de gants, doigtiers ou préservatifs revêtus d'un film d'élastomère conforme à l'invention.

En tant que revêtement de matériau élastomère, le film d'élastomère conforme à l'invention est rapporté sur une couche de matériau élastomère préalablement façonnée à la forme voulue par les techniques usuelles de fabrication, comme suit :

On dépose sur une forme en céramique, en verre ou en matériau analogue définissant le gant, le doigtier ou le préservatif à fabriquer, une couche d'élastomère, conformément à la Demande EP 306 389.

La forme revêtue de ladite première couche est alors soumise à un premier traitement de pré-vulcanisation, puis en ce qu'après immersion dans une émulsion stabilisée conforme à l'invention, on vulcanise l'ensemble qui est ensuite retiré de la forme.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Film d'élastomère comprenant une substance chimique active sous forme liquide, lequel film est caractérisé en ce qu'il comprend :
- une dispersion de gouttelettes consistant en une solution, dans un polyol, d'une substance chimique active sélectionnée dans le groupe constitué par les anticorrosifs, les lubrifiants et les biocides, et
- un copolymère de stabilisation de ladite dispersion de gouttelettes comportant au moins des séquences poly B, miscibles avec lesdites gouttelettes, et des séquences poly A, non miscibles avec ces gouttelettes, ledit copolymère étant sélectionné dans le groupe constitué par les copolymères di-bloc, de type poly A-bloc-poly B, les copolymères tri-bloc sélectionnés dans le groupe constitué par les copolymères poly B-bloc-poly A-bloc-poly B (BAB), poly A-bloc-poly B-bloc-poly A (ABA), poly A-bloc-poly B-bloc-poly C (ABC) et poly A-bloc-poly C-bloc-poly B (ACB) et les copolymères greffés de type poly A-greffé-poly B, poly B-greffé-poly A, de type poly A-greffé-poly B et poly C ou de type poly C-greffé-poly A et poly B.

2. Film d'élastomère selon la revendication 1, caractérisé en ce que le polyol est sélectionné dans le groupe constitué par le polypropylène glycol, le polyéthylène glycol et le glycérol et un mélange de ceux-ci.

3. Film d'élastomère selon la revendication 1 ou la revendication 2, caractérisé en ce que les proportions de séquences poly A sont comprises entre 10 et 90 % et les proportions de séquences poly B sont comprises entre 90 et 10 %, par rapport à la somme des séquences poly A + poly B et les proportions de séquences poly C sont comprises entre 0 % et 50 %, par rapport au total de séquences.

4. Film d'élastomère selon l'une quelconque des revendications 1 à 3, caractérisé en ce que les masses moléculaires des séquences poly A et poly B sont comprises entre 1 000 et 500 000 Daltons.

5. Film d'élastomère selon l'une quelconque des revendications 1 à 4, caractérisé en ce que les séquences poly A sont choisies dans le groupe constitué par les polydiènes, les polyoléfines, les polyéthers ou les silicones miscibles avec une solution d'élastomère dans un solvant a apolaire ou peu polaire et non-miscibles avec le polyol, et les séquences poly B, miscibles avec le polyol sont choisies dans le groupe constitué par le polyoxyéthylène, la polyvinylpyrrolidone, les polyacides acryliques, le poly(alcool vinylique) et le poly(vinylpyridine) quaternisé.

6. Film d'élastomère selon la revendication 5, caractérisé en ce que les séquences poly A sont sélectionnées dans le groupe constitué par le polyisoprène, le polybutadiène, le polyisobutène, le polybutadiène hydrogéné ou le polyisoprène hydrogéné, le polystyrène, le polytertiobutylstyrène, le polyoxypropylène, le polydiméthylsiloxane.

7. Film d'élastomère selon l'une quelconque des revendications 1 à 6, caractérisé en ce qu'il est sélectionné dans le groupe constitué par le polybutadiène, le polyisoprène, le polychloroprène, les copolymères SBR *(Styrene Butadiene Rubber)*, NBR (*Nitrile Butadiene Rubber*), SBS *(Styrene Butadiene Styrene)* ou SIS *(Styrene Isoprene Styrene)* ou SEBS, de masse moléculaire de préférence supérieure à 50 000.

8. Film d'élastomère selon l'une quelconque des revendications 1 à 7, caractérisé en ce que la substance chimique active est choisie parmi les biocides.

9. Film d'élastomère selon la revendication 8, caractérisé en ce que ledit biocide est sélectionné dans le groupe constitué par les ammonium quaternaires, les copolymères bloc à activité biocide, les biguanides, le phtaraldéhyde, les dérivés phénoliques à activité biocide, les tensioactifs non ioniques comportant une séquence polyoxyéthylène ou l'hexamidine et leur mélange.

10. Film d'élastomère selon l'une quelconque des revendications 1 à 7, caractérisé en ce que la substance chimique active est choisie dans le groupe constitué par les anticorrosifs et les lubrifiants solubles dans les polyols.

11. Procédé de préparation d'un film d'élastomère, caractérisé en ce qu'il comprend :
(a) la préparation d'une émulsion qui comprend :
- la préparation d'une phase A par dissolution de l'élastomère dans un solvant organique a ;
- la préparation d'une phase B, par mélange d'au moins une substance chimique active sélectionnée dans le groupe constitué par les anticorrosifs, les lubrifiants et les biocides, dans un solvant organique b consistant en un polyol, non miscible avec la phase A ;
- l'addition à la phase A ou à la phase B, dans des proportions de 0,1 à 50 %, de préférence de 0,1 à 25 %, d'un copolymère bloc ou greffé de stabilisation comportant au moins des séquences poly A et au moins des séquences poly B, ledit copolymère étant sélectionné dans le groupe constitué par les copolymères di-bloc, de type poly A-bloc-poly B, les copolymères tri-bloc sélectionnés dans le groupe constitué par les copolymères poly B-bloc-poly A-bloc-poly B (BAB), poly A-bloc-poly B-bloc-poly A (ABA), poly A-bloc-poly B-bloc-poly C (ABC) et poly A-bloc-poly C-bloc-poly B (ACB) et les copolymères greffés de type poly A-greffé-poly B, poly B-greffé-poly A, de type poly A-greffé-poly B et poly C ou de type poly C-greffé-poly A et poly B, dans lesquels les séquences poly A sont miscibles avec la phase A et sont sélectionnées dans le groupe constitué par les polydiènes, les polyoléfines, les polyéthers et les silicones, les séquences poly B sont miscibles avec la phase B et sont sélectionnées dans le groupe constitué par le polyoxyéthylène, la polyvinylpyrrolidone, les polyacides acryliques, le poly(alcool vinylique) et le poly(vinylpyridine) quaternisé et lesdites séquences poly C sont soit miscibles avec le solvant a, soit avec le polyol, soit non miscibles avec le solvant a et le polyol ;
- la dispersion de la phase B dans la phase A, pour l'obtention d'une émulsion dans laquelle la phase A constitue la phase continue et la phase B, la phase dispersée ; et
(b) l'évaporation du solvant organique a, pour l'obtention d'un film d'élastomère renfermant sous forme de dispersion stable, des gouttelettes de ladite substance chimique active dans un polyol.

12. Procédé selon la revendication 11, caractérisé en ce que le solvant organique apolaire ou peu polaire a est choisi dans le groupe constitué par les hydrocarbures aromatiques, aliphatiques et alicycliques, par exemple des hydrocarbures paraffiniques, le cyclohexane, le benzène, le toluène, le xylène, la tétraline, la décaline ou un mélange de ceux-ci.

13. Procédé selon la revendication 11 ou la revendication 12, caractérisé en ce que le polyol est sélectionné dans le groupe constitué par le polypropylène glycol, le polyéthylène glycol et le glycérol ou des mélanges de ceux-ci.

14. Procédé selon l'une quelconque des revendications 11 à 13, caractérisé en ce que les proportions de séquences poly A sont comprises entre 10 et 90 % et les proportions de séquences poly B sont comprises entre 90 et 10 %, par rapport à la somme de séquences poly A + poly B et les proportions de séquences poly C sont comprises entre 0 % et 50 % par rapport au total de séquences.

15. Procédé selon l'une quelconque des revendications 11 à 14, caractérisé en ce que les masses moléculaires des séquences poly A et poly B sont comprises entre 1 000 et 500 000 Daltons.

16. Emulsion apte à être utilisée pour la préparation d'un film d'élastomère, caractérisée en ce qu'elle comprend :
- une phase A comprenant un élastomère dissous dans un solvant organique a tel que défini à la revendication 12, dans laquelle est dispersée une phase B comprenant au moins une substance chimique active sélectionnée dans le groupe constitué par les anticorrosifs, les lubrifiants et les biocides, en solution ou dispersée dans un solvant b consistant en un polyol non miscible avec la phase A, tel que défini à la revendication 13,
- et un copolymère bloc ou greffé comportant au moins des séquences poly A et au moins des séquences poly B, ledit copolymère étant sélectionné dans le groupe constitué par les copolymères di-bloc, de type poly A-bloc-poly B, les copolymères tri-bloc sélectionnés dans le groupe constitué par les copolymères poly B-bloc-poly A-bloc-poly B (BAB), poly A-bloc-poly B-bloc-poly A (ABA), poly A-bloc-poly B-bloc-poly C (ABC) et poly A-bloc-poly C-bloc-poly B (ACB) et les copolymères greffés de type poly A-greffé-poly B, poly B-greffé-poly A, de type poly A-greffé-poly B et poly C ou de type poly C-greffé-poly A et poly B, dans lesquels les séquences poly A sont miscibles avec la phase A et sont sélectionnées dans le groupe constitué par les polydiènes, les polyoléfines, les polyéthers et les silicones, les séquences poly B sont miscibles avec la phase B et sont sélectionnées dans le groupe constitué par le polyoxyéthylène, la polyvinylpyrrolidone, les polyacides acryliques, le poly(alcool vinylique) et le poly(vinylpyridine) quaternisé et lesdites séquences poly C sont soit miscibles avec le solvant a, soit avec le polyol, soit non miscibles avec le solvant a et le polyol.

17. Procédé de préparation desdites émulsions stables selon la revendication 16, caractérisé en ce qu'il comprend :
- la préparation d'une phase A par dissolution de l'élastomère dans un solvant organique a ;
- la préparation d'une phase B par mélange d'au moins une substance chimique active sélectionnée dans le groupe constitué par les anticorrosifs, les lubrifiants et les biocides, dans un solvant organique b constitué par un polyol, non miscible avec la phase A ;
- l'addition à la phase A ou à la phase B, dans des proportions de 0,1 à 50 %, de préférence de 0,1 à 25 %, d'un copolymère bloc ou greffé, comportant au moins des séquences poly A, miscibles avec la phase A et des séquences poly B, miscibles avec la phase B ; et
- la dispersion de la phase B dans la phase A pour l'obtention d'une émulsion dans laquelle la phase A constitue la phase continue et la phase B, la phase dispersée.

18. Application du film d'élastomère selon l'une quelconque des revendications 1 à 10 en tant que revêtement de supports.

19. Gant, caractérisé en ce qu'il est revêtu d'un film d'élastomère selon l'une quelconque des revendications 1 à 9.

20. Doigtier, caractérisé en ce qu'il est revêtu d'un film d'élastomère selon l'une quelconque des revendications 1 à 9.

21. Préservatif, caractérisé en ce qu'il est revêtu d'un film d'élastomère selon l'une quelconque des revendications 1 à 9.

22. Joint frottant, caractérisé en ce qu'il est revêtu d'un film d'élastomère selon l'une quelconque des revendications 1 à 7 ou 10.

23. Pansement, caractérisé en ce qu'il est revêtu d'un film d'élastomère selon l'une quelconque des revendications 1 à 9.

## Claims

1. Elastomer film including an active chemical substance in liquid form, which film is characterized in that it includes:
- a dispersion of droplets consisting of a solution, in a polyol, of an active chemical substance selected in the group consisting of anticorrosive agents, lubricants and biocides, and
- a stabilizing copolymer for said dispersion of droplets comprising at least polyB blocks which are miscible with the said droplets and polyA blocks which are not miscible with said droplets, said copolymer being selected in the group consisting of diblock copolymers of polyA-block-polyB type, triblock copolymers of polyB-block-polyA-block-polyB (BAB) type, of polyA-block-polyB-block-polyA (ABA) type, of polyA-block-polyB-block-polyC (ABC) or polyA-block-polyC-block-polyB (ACB) type and graft copolymers of polyA-graft-polyB type, of polyB-graft-polyA type, of polyA-graft-polyB and polyC type or of polyC-graft-polyA and polyB type.

2. Elastomer film according to Claim 1, characterized in that the said polyol is selected in the group consisting of polypropylene glycol, polyethylene glycol and glycerol and a mixture therefrom.

3. Elastomer film according to Claim 1 or Claim 2, characterized in that the proportions of polyA blocks are between 10 and 90 % and the proportions of polyB blocks are between 90 and 10 %, relative to the sum of the polyA + polyB blocks, and the proportions of polyC blocks are between 0 % and 50 % relative to the total of blocks.

4. Elastomer film according to any one of Claims 1 to 3, characterized in that the molecular masses of the polyA and polyB blocks are between 1 000 and 500 000 daltons.

5. Elastomer film according to any one of Claims 1 to 4, characterized in that the polyA blocks are selected from the group consisting of polydienes, polyolefins, polyethers or silicones which are miscible with a solution of elastomer in an apolar or weakly polar solvent a, and not miscible with the polyol, and the polyB blocks, which are miscible with the polyol, are chosen from the group consisting of polyoxyethylene, polyvinylpyrrolidone, polyacrylic acids, poly(vinyl alcohol) and quaternized poly(vinylpyridine).

6. Elastomer film according to Claim 5, characterized in that the polyA blocks are selected in the group consisting of polyisoprene, polybutadiene, polyisobutene, hydrogenated polybutadiene or hydrogenated polyisoprene, polystyrene, poly-tert-butylstyrene, polyoxypropylene and polydimethylsiloxane.

7. Elastomer film according to any one of Claims 1 to 6, characterized in that it is selected in the group consisting of polybutadiene, polyisoprene, polychloroprene, SBR *(styrene butadiene rubber)*, NBR *(nitrile butadiene rubber)*, SBS *(styrene butadiene styrene)* or SIS *(styrene isoprene styrene)* or SEBS copolymers, of molecular mass which is preferably higher than 50 000.

8. Elastomer film according to any one of Claims 1 to 7, characterized in that the active chemical substance is chosen from biocides.

9. Elastomer film according to Claim 8, characterized in that the said biocide is selected in the group consisting of quaternary ammoniums, block copolymers with biocidal activity, biguanides, phthalaldehyde, phenolic derivatives with biocidal activity, nonionic surfactants containing a polyoxyethylene block or hexamidine, and a mixture therefrom.

10. Elastomer film according to any one of Claims 1 to 7, characterized in that the active chemical substance is chosen from the group consisting of the anticorrosive agents and the lubricants which are soluble in polyols.

11. Process for the preparation of an elastomer film, characterized in that it includes:
(a) the preparation of an emulsion which includes:
- the preparation of a phase A by dissolving the elastomer in an organic solvent a;
- the preparation of a phase B, by mixing at least one active substance selected in the group consisting of anticorrosive agents, lubricants and biocides, into an organic solvent b consisting of a polyol, which is not miscible with the phase A;
- the addition to the phase A or to the phase B, in proportions of 0.1 to 50 %, preferably of 0.1 to 25 %, of a block or graft stabilizing copolymer comprising at least polyA blocks and at least polyB blocks, said copolymer being selected in the group consisting of diblock copolymers of polyA-block-polyB type, triblock copolymers of polyB-block-polyA-block-polyB (BAB) type, of polyA-block-polyB-block-polyA (ABA) type, of polyA-block-polyB-block-polyC (ABC) or polyA-block-polyC-block-polyB (ACB) type and graft copolymers of polyA-graft-polyB type, of polyB-graft-polyA type, of polyA-graft-polyB and polyC type or of polyC-graft-polyA and polyB type, wherein the polyA blocks are miscible with the phase A and are selected from the group consisting of polydienes, polyolefins, polyethers or silicones, the polyB bloks are miscible with the phase B and are chosen from the group consisting of polyoxyethylene, polyvinylpyrrolidone, polyacrylic acids, poly(vinyl alcohol) and quaternized poly(vinylpyridine), and said polyC blocks are either miscible with the solvent a, or with the polyol, or not miscible with the solvent a and the polyol;
- the dispersion of the phase B in the phase A in order to obtain an emulsion in which the phase A forms the continuous phase and the phase B the disperse phase; and
(b) the evaporation of the organic solvent a, in order to obtain an elastomer film containing, in the form of a stable dispersion, droplets of said active chemical substance in a polyol.

12. Process according to Claim 11, characterized in that the apolar or weakly polar organic solvent a, is chosen from the group consisting of aromatic, aliphatic and alicyclic hydrocarbons, for example paraffinic hydrocarbons, cyclohexane, benzene, toluene, xylene, tetralin, decalin or a mixture thereof.

13. Process according to Claim 11 or Claim 12, characterized in that the polyol is selected in the group consisting of polypropylene glycol, polyethylene glycol and glycerol or mixtures thereof.

14. Process according to any one of Claims 11 to 13, characterized in that the proportions of polyA blocks are between 10 and 90 % and the proportions of polyB blocks are between 90 and 10 %, relative to the sum of polyA + polyB blocks, and the proportions of polyC blocks are between 0 % and 50 % relative to the total of blocks.

15. Process according to any one of Claims 11 to 14, characterized in that the molecular masses of the polyA and polyB blocks are between 1 000 and 500 000 daltons.

16. Emulsion capable of being employed for the preparation of an elastomer film, characterized in that it includes:
- a phase A including an elastomer dissolved in an organic solvent a as defined in Claim 12, in which is dispersed a phase B including at least one active chemical substance selected in the group consisting of anticorrosive agents, lubricants and biocides, in solution or dispersed in a solvent b consisting of a polyol which is not miscible with the phase A, as defined in Claim 13,
- and a block or graft copolymer comprising at least polyA blocks and at least polyB blocks, said copolymer being selected in the group consisting of diblock copolymers of polyA-block-polyB type, triblock copolymers of polyB-block-polyA-block-polyB (BAB) type, of polyA-block-polyB-block-polyA (ABA) type, of polyA-block-polyB-block-polyC (ABC) or polyA-block-polyC-block-polyB (ACB) type and graft copolymers of polyA-graft-polyB type, of polyB-graft-polyA type, of polyA-graft-polyB and polyC type or of polyC-graft-polyA and polyB type, wherein the **polyA blocks** are miscible with the phase A and are selected in the group consisting of polydienes, polyolefins, polyethers or silicones, the **polyB bloks** are miscible with the phase B and are chosen from the group consisting of polyoxyethylene, polyvinylpyrrolidone, polyacrylic acids, poly(vinyl alcohol) and quaternized poly(vinylpyridine), and said polyC blocks are either miscible with the solvent a, or with the polyol, or not miscible with the solvent a and the polyol.

17. Process for the preparation of the said stable emulsions according to Claim 16, characterized in that it includes:
- the preparation of a phase A by dissolving the elastomer in an organic solvent a;
- the preparation of a phase B by mixing at least one active substance selected in the group consisting of anticorrosive agents, lubricants and biocides into an organic solvent b consisting of a polyol which is not miscible with the phase A;
- the addition to the phase A or to the phase B, in proportions of 0.1 to 50 %, preferably of 0.1 to 25 %, of a block or graft copolymer comprising at least polyA blocks which are miscible with the phase A and polyB blocks which are miscible with the phase B; and
- the dispersion of the phase B in the phase A in order to obtain an emulsion in which the phase A forms the continuous phase and the phase B the disperse phase.

18. Application of the elastomer film according to any one of Claims 1 to 10 as coating for supports.

19. Glove, characterized in that it is coated with an elastomer film according to any one of Claims 1 to 9.

20. Fingerstall, characterized in that it is coated with an elastomer film according to any one of Claims 1 to 9.

21. Protective sheath, characterized in that it is coated with an elastomer film according to any one of Claims 1 to 9.

22. Rubbing seal, characterized in that it is coated with an elastomer film according to any one of Claims 1 to 7 or 10.

23. Dressing, characterized in that it is coated with an elastomer film according to any one of Claims 1 to 9.

## Patentansprüche

1. Elastomerfilm, umfassend eine aktive chemische Substanz in flüssiger Form, dadurch **gekennzeichnet,**
daß er folgendes umfaßt:
- eine Dispersion von Tröpfchen, bestehend aus einer Lösung in einem Polyol, einer aktiven chemischen Substanz, ausgewählt aus der Gruppe bestehend aus Antikorrosionsmitteln, Schmiermitteln und Biociden, und
- ein Copolymer zur Stabilisierung der genannten Dispersion von Tröpfchen, umfassend mindestens poly-B-Sequenzen, die mit den genannten Tröpfchen mischbar sind, und poly-A-Sequenzen, die mit diesen Tröpfchen nicht mischbar sind, wobei das genannte Copolymer ausgewählt ist aus der Gruppe bestehend aus Di-Block-Copolymeren vom Typ poly-A-Block-poly-B, Tri-Block-Copolymeren, ausgewählt aus der Gruppe bestehend aus Copolymeren poly-B-Block-poly-A-Block-poly-B (BAB), poly-A-Block-poly-B-Block-poly-A (ABA), poly-A-Block-poly-B-Block-poly-C (ABC) und poly-A-Block-poly-C-Block-poly-B (ACB) und Pfropf-Copolymeren vom Typ poly A-Pfropf-poly-B, poly B-Pfropt-poly-A, vom Typ poly-A-Pfropf-poly-B und -poly-C oder vom Typ poly-C-Pfropf-poly-A und -poly-B.

2. Elastomerfilm nach Anspruch 1, dadurch **gekennzeichnet,** daß das Polyol ausgewählt ist aus der Gruppe bestehend aus Polypropylenglycol, Polyethylenglycol und Glycerin und einem Gemisch derselben.

3. Elastomerfilm nach Anspruch 1 oder 2, dadurch **gekennzeichnet,** daß die Verhältnisse der poly-A-Sequenzen zwischen 10 und 90 % liegen und die Verhältnisse der poly-B-Sequenzen zwischen 90 und 10 % liegen, bezogen auf die Summe der poly-A- + poly-B-Sequenzen und die Verhältnisse der poly-C-Sequenzen zwischen 0 % und 50 % liegen, bezogen auf die Gesamtmenge der Sequenzen.

4. Elastomerfilm nach einem der Ansprüche 1 bis 3, dadurch **gekennzeichnet,** daß die Molekularmassen der poly-A- und poly-B-Sequenzen zwischen 1.000 und 500.000 Dalton liegen.

5. Elastomerfilm nach einem der Ansprüche 1 bis 4, dadurch **gekennzeichnet,** daß die poly-A-Sequenzen ausgewählt sind aus der Gruppe bestehend aus Polydienen, Polyolefinen, Polyethern oder Siliconen, die mit einer Elastomerlösung in einem apolaren oder wenig polaren Lösungsmittel a mischbar und mit dem Polyol nicht mischbar sind und die mit dem Polyol mischbaren poly-B-Sequenzen ausgewählt sind aus der Gruppe bestehend aus Polyoxyethylen, Polyvinylpyrrolidon, Polyacrylsäuren, Poly(vinylalkohol) und quaternisiertem Poly(vinylpyridin).

6. Elastomerfilm nach Anspruch 5, dadurch **gekennzeichnet,** daß die poly-A-Sequenzen ausgewählt sind aus der Gruppe bestehend aus Polyisopren, Polybutadien, Polyisobuten, hydriertem Polybutadien oder hydriertem Polyisopren, Polystyrol, Polytertbutylstyrol, Polyoxypropylen, Polydimethylsiloxan.

7. Elastomerfilm nach einem der Ansprüche 1 bis 6, dadurch **gekennzeichnet,** daß er ausgewählt ist aus der Gruppe bestehend aus Polybutadien, Polyisopren, Polychloropren, SBR-Copolymeren (Styrene Butadiene Rubber), NBR-Copolymeren (Nitrile Butadiene Rubber), SBS-Copolymeren (Styrene Butadiene Styrene) oder SIS-Copolymeren (Styrene Isoprene Styrene) oder SEBS mit einer Molekularmasse von vorzugsweise mehr als 50.000.

8. Elastomerfilm nach einem der Ansprüche 1 bis 7, dadurch **gekennzeichnet,** daß die aktive chemische Substanz ausgewählt ist aus Biociden.

9. Elastomerfilm nach Anspruch 8, dadurch **gekennzeichnet,** daß das genannte Biocid ausgewählt ist aus der Gruppe bestehend aus quaternären Ammoniumverbindungen, Block-Copolymeren mit biocider Aktivität, Biguaniden, Phtharaldehyd, phenolischen Derivaten mit biocider Aktivität, nichtionischen oberflächenaktiven Stoffen, die eine Polyoxyethylensequenz umfassen, oder Hexamidin und deren Gemisch.

10. Elastomerfilm nach einem der Ansprüche 1 bis 7, dadurch **gekennzeichnet,** daß die aktive chemische Substanz ausgewählt ist aus der Gruppe bestehend aus Antikorrosionsmitteln und Schmiermitteln, die in Polyolen löslich sind.

11. Verfahren zur Herstellung eines Elastomerfilms, dadurch **gekennzeichnet,** daß es folgendes umfaßt:
(a) Die Herstellung einer Emulsion, die umfaßt:
- die Herstellung einer Phase A durch Auflösen des Elastomers in einem organischen Lösungmittel a;
- die Herstellung einer Phase B durch Einmischen von mindestens einer aktiven chemischen Substanz, ausgewählt aus der Gruppe bestehend aus Antikorrosionsmitteln, Schmiermitteln und Biociden, in ein organisches Lösungsmittel b, bestehend aus einem Polyol, das nicht mit der Phase A mischbar ist;
- die Zugabe zu der Phase A oder zu der Phase B in Verhältnissen von 0,1 bis 50 %, bevorzugt 0,1 bis 25 %, eines Block- oder Pfropf-Copolymers zur Stabilisierung, umfassend mindestens poly-A-Sequenzen und mindestens poly-B-Sequenzen, hobei das genannte Copolymer ausgewählt ist aus der Gruppe bestehend aus Di-Block-Copolymeren vom Typ poly-A-Block-poly-B, den Tri-Block-Copolymeren, ausgewählt aus der Gruppe bestehend aus den Copolymeren poly-B-Block-poly-A-Block-poly-B (BAB), poly-A-Block-poly-B-Block-poly-A(ABA), poly-A-Block-poly-B-Block-poly-C (ABC) und poly-A-Block-poly-C-Block-poly-B (ACB) und Pfropf-Copolymeren vom Typ poly-A-Pfropf-poly-B, poly-B-Pfropf-poly-A, vom Typ poly-A-Pfropf-poly-B und -poly-C oder vom Typ poly-C-Pfropf-poly-A und -poly-B, in denen die poly-A-Sequenzen mit der Phase A mischbar sind und ausgewählt sind aus der Gruppe bestehend aus Polydienen, Polyolefinen, Polyethern und Siliconen, die poly-B-Sequenzen mit der Phase B mischbar sind und ausgewählt sind aus der Gruppe bestehend aus Polyoxyethylen, Polyvinylpyrrolidon, Polyacrylsäuren, Poly(vinylalkohol) und quaternisiertem Poly(vinylpyridin) und die genannten poly-C-Sequenzen, die entweder mit dem Lösungsmittel a oder dem Polyol mischbar sind oder mit dem Lösungsmittel a und dem Polyol nicht mischbar sind;
- das Dispergieren der Phase B in der Phase A, um eine Emulsion zu erhalten, in der die Phase A die kontinuierliche Phase und die Phase B die dispergierte Phase bildet; und
(b) das Verdampfen des organischen Lösungsmittels a, um einen Elastomerfilm zu erhalten, der in Form einer stabilen Dispersion Tröpfchen der genannten aktiven chemischen Substanz in einem Polyol enthält.

12. Verfahren nach Anspruch 11, dadurch **gekennzeichnet,** daß das organische apolare oder wenig polare Lösungsmittel a ausgewählt ist aus der Gruppe bestehend aus aromatischen, aliphatischen und alicyclischen Kohlenwasserstoffen, z.B. paraffinischen Kohlenwasserstoffen, Cyclohexan, Benzol, Toluol, Xylol, Tetralin, Decalin oder einem Gemisch derselben.

13. Verfahren nach Anspruch 11 oder 12, dadurch **gekennzeichnet,** daß das Polyol ausgewählt ist aus der Gruppe bestehend aus Polypropylenglycol, Polyethylenglycol und Glycerin oder Gemischen derselben.

14. Verfahren nach einem der Ansprüche 11 bis 13, dadurch **gekennzeichnet,** daß die Verhältnisse der poly-A-Sequenzen zwischen 10 und 90 % liegen und die Verhältnisse der poly-B-Sequenzen zwischen 90 und 10 % liegen, bezogen auf die Summe der poly-A- + poly-B-Sequenzen, und die Verhältnisse der poly-C-Sequenzen zwischen 0 % and 50 % liegen, bezogen auf die Gesamtmenge der Sequenzen.

15. Verfahren nach einem der Ansprüche 11 bis 14, dadurch **gekennzeichnet,** daß die Molekularmassen der poly-A- und poly-B-Sequenzen zwischen 1.000 und 500.000 Dalton liegen.

16. Emulsion, die geeignet ist, zur Herstellung eines Elastomerfilms verwendet zu werden, dadurch **gekennzeichnet,** daß sie folgendes umfaßt:
eine Phase A, umfassend ein Elastomer, das in einem organischen Lösungsmittel a, wie in Anspruch 12 definiert, gelöst ist, in der eine Phase B dispergiert ist, die mindestens eine aktive chemische Substanz, ausgewählt aus der Gruppe bestehend aus Antikorrosionsmitteln, Schmiermitteln und Biociden, umfaßt, in Lösung oder dispergiert in einem Lösungsmittel b, bestehend aus einem mit der Phase A nicht mischbaren Polyol, wie in Anspruch 13 definiert,
und ein Block- oder Pfropf-Copolymer, umfassend mindestens poly A Sequenzen und mindestens poly-B-Sequenzen, wobei das genannte Copolymer ausgewählt ist aus der Gruppe bestehend aus Di-Block-Copolymeren vom Typ poly-A-Block-poly-B, Tri-Block-Copolymeren, ausgewählt aus der Gruppe bestehend aus Copolymeren poly-B-Block-poly-A-Block-poly-B (BAB), poly-A-Block-poly-B-Block-poly-A (ABA), poly-A-Block-poly-B-Block-poly-C (ABC) und poly-A-Block-poly-C-Block-poly-B (ACB) und Pfropf-Copolymeren vom Typ poly-A-Pfropf-poly-B, poly-B-Pfropf-poly-A, vom Typ poly-A-Pfropf-poly-B und -poly-C oder vom Typ poly-C-Pfropf-poly-A und -poly-B, in denen die poly-A-Sequenzen mit der Phase A mischbar sind und ausgewählt sind aus der Gruppe bestehend aus Polydienen, Polyolefinen, Polyethern und Siliconen, die poly B Sequenzen mit der Phase B mischbar sind, und ausgewählt sind aus der Gruppe bestehend aus Polyoxyethylen, Polyvinylpyrrolidon, Polyacrylsäuren, poly(vinylalkohol) und quaternisiertem poly(vinylpyridin) und die genannten poly-C-Sequenzen entweder mit dem Lösungsmittel a oder dem Polyol mischbar sind oder nicht mit dem Lösungsmittel a und dem Polyol mischbar sind.

17. Verfahren zur Herstellung der genannten stabilen Emulsionen nach Anspruch 16, dadurch **gekennzeichnet,**
daß es folgendes umfaßt:
- die Herstellung einer Phase A durch Auflösen des Elastomers in einem organischen Lösungsmittel a;
- die Herstellung einer Phase B durch Einmischen von mindenstens einer aktiven chemischen Substanz, ausgewählt aus der Gruppe bestehend aus Antikorrosionsmitteln, Schmiermitteln und Biociden in ein organisches Lösungsmittel b, bestehend aus einem Polyol, das nicht mit der Phase A mischbar ist;
- das Zugeben zu der Phase A oder zu der Phase B in den Verhältnissen von 0,1 bis 50 %, bevorzugt 0,1 bis 25 %, eines Block- oder Pfropf-Copolymers, umfassend mindestens poly-A-Sequenzen, die mit der Phase A mischbar sind, und poly-B-Sequenzen, die mit der Phase B mischbar sind; und
- das Dispergieren der Phase B in der Phase A, um eine Emulsion zu erhalten, in der die Phase A die kontinuierliche Phase und die Phase B die dispergierte Phase bildet.

18. Verwendung eines Elastomerfilms nach einem der Ansprüche 1 bis 10 als Belag bzw. Überzug von Trägermaterialien.

19. Handschuh, dadurch **gekennzeichnet,** daß er mit einen Elastomerfilm nach einem der Ansprüche 1 bis 9 belegt bzw. überzogen ist.

20. Fingerling, dadurch **gekennzeichnet,** daß er mit einem Elastomerfilm nach einem der Ansprüche 1 bis 9 belegt bzw. überzogen ist.

21. Präservativ, dadurch **gekennzeichnet,** daß es mit einem Elastomerfilm nach einem der Ansprüche 1 bis 9 belegt bzw. überzogen ist.

22. Reibende Dichtung bzw. Verbindung, dadurch **gekennzeichnet,** daß sie mit einem Elastomerfilm nach einem der Ansprüche 1 bis 7 oder 10 belegt bzw. überzogen ist.

23. Verband, dadurch **gekennzeichnet,** daß er mit einem Elastomerfilm nach einem der Ansprüche 1 bis 9 belegt bzw. überzogen ist.
